Europäisches Patentamt

European Patent Office

Office européen des brevets

(11) Publication number: **0 291 594 B1**

# EUROPEAN PATENT SPECIFICATION

(45) Date of publication of patent specification: **07.08.91**   (51) Int. Cl.5: **A61K 31/495**

(21) Application number: **87304503.3**

(22) Date of filing: **20.05.87**

(54) **Pyrazine for use in the treatment of haemodynamic and metabolic disorders.**

(43) Date of publication of application:
**23.11.88 Bulletin 88/47**

(45) Publication of the grant of the patent:
**07.08.91 Bulletin 91/32**

(84) Designated Contracting States:
**DE ES FR IT**

(56) References cited:
**US-A- 3 720 768**

**JOURNAL OF THE AMERICAN CHEMICAL SO-
CIETY, vol. 74, 1952, pages 1580-1584, US; G.
KARMAS et al.: "The preparation of hydrox-
ypyrazines and derived chloropyrazines"**

(73) Proprietor: **TOYO JOZO KABUSHIKI KAISHA**
**632-1 Mifuku Ohito-cho**
**Tagata-gun Shizuoka-ken(JP)**

(72) Inventor: **Hayashi, Eiichi**
**7-32, Midori-cho**
**Shizuoka-shi Shizuoka-ken(JP)**
Inventor: **Takayanagi, Noriyasu**
**284-2, Tokura Shimizu-cho**
**Sunto-gun Shizuoka-ken(JP)**
Inventor: **Nishimura, Katsumi**
**632-1, Mifuku Ohito-cho**
**Tagata-gun Shizuoka-ken(JP)**
Inventor: **Yaso, Masao**
**848-1, Takyo Ohito-cho**
**Tagata-gun Shizuoka-ken(JP)**
Inventor: **Suzuki, Yukio**
**525-1, Mifuku Ohito-cho**
**Tagata-gun Shizuoka-ken(JP)**

(74) Representative: **Myerscough, Philip Boyd et al**
**J.A.Kemp & Co. 14, South Square Gray's Inn**
**London, WC1R 5EU(GB)**

**Description**

This invention relates to an agent for improving haemodynamic and metabolic disordes.

Recently, there have been reports on compounds having an inhibitory effect on platelet aggregation. Among these compounds, ones having a pyrazine, ring as a main nucleus have not been much reported. Only the compound tetramethylpyrazine [Abst. of the 16th congress of heterocyclic chemistry (Osaka) p. 65-68 (1984)] and 2-higher fatty acyloxymethyl pyrazines (JP-A-59-219269) are known.

We have synthesised various kinds of pyrazine derivatives and have screened their pharmacological activities. We have found that 2-hydroxy-3-isopropyl-5,6-dimethylpyrazine indicated an inhibitory effect on platelet aggregation, increasing effect on blood flow for organs and tissues and/or protective effect against anoxia which reveal advantageous pharmacological activities. 2-Hydroxy-3-isopropyl-5,6-dimethylpyrazine is a known compound. However pharmacological activity-thereof has not been known.

J. Am. Chem. Soc., 74:1580-1584(1952), G.Karmas et al, describes the preparation of various hydroxypyrazines from ∝-dicarbonylcompounds and hydrohalides of aminoacid amides. 2-hydroxy-3-isopropyl-5,6-dimethylpyrazine is described, but no possible therapeutic activity is mentioned.

US-A-3720768 discloses that aspergillic acid and some derivatives thereof can be used to treat hypertension. However the derivatives described do not include 2-hydroxy-3-isoproyl-5,6-dimethylpyrazine.

The present invention provides 2-hydroxy-3-isopropyl-5,6-dimethylpyrazine or a non-toxic salt thereof for use in a method of treatment of the human or animal body by therapy. The compounds may be used in the treatment of haemodynamic and metabolic disorders. For example, they may be used in inhibiting platelet aggregation, as a vasodilator and in protecting against anoxia. The preferred salt is the hydrochloride.

The invention further provides the use of 2-hydroxy-3-isopropyl-5,6-dimethylpyrazine or a non-toxic salt thereof in the preparation of a pharmaceutical composition for use in a method of treatment of the human or animal body by therapy, especially for use in a method of treatment of a haemodynamic or metabolic disorder. Also provided are pharmaceutical compositions comprising 2-hydroxy-3-isopropyl-5,6-dimethylpyrazine or a non-toxic salt thereof as active ingredient and a pharmaceutically acceptable carrier or diluent, and a process for the preparation of a pharmaceutical composition, which process comprises mixing 2-hydroxy-3-isopropyl-5,6-dimethylpyrazine or a non-toxic salt thereof with a pharmaceutically acceptable carrier or diluent, the said mixing being effected under sterile conditions and/or the obtained mixture being sterilised.

Also part of the invention of non-toxic salts of 2-hydroxy-3-isopropyl-5,6-dimethylpyrazine. These are prepared by a process comprising condensing 2,3-butanedione with valineamide in the presence of a tertiary amine and converting the resultant 2-hydroxy-3-isopropyl-5,6-dimethylpyrazine into a non-toxic salt thereof.

Examples of non-toxic salts are pharmacologically acceptable salts, for example inorganic or organic salts such as the hydrochloride, sulfate, carbonate, nitrate, hydrobromide, phosphate, sulfonate, acetate, oxalate, tartrate, citrate, malate, glutamate or aspartate.

The 2-hydroxy-3-isopropyl-5,6-dimethylpyrazine or non-toxic salt thereof can be a hydrate. The hydrate is also included in the present invention.

The 2-hydroxy-3-isopropyl-5,6-dimethylpyrazine can be produced according to the general principles of the method disclosed in Reuben G. Jones; J. Am. Chem. Soc., 71: 78-81 (1949) , namely by condensing 2,3-butanedione with valineamide, a type of α-amino acidamide in the presence of a tertiary amine such as triethylamine.

The pharmacological activities and toxicities of 2-hydroxy-3-isopropyl-5,6-dimethylpyrazine hydrochloride are now illustrated.

Test Example 1

Inhibitory effect on platelet aggregation:

(1) Effect in vitro :

Solution of agents to be tested (final concentration 100 μM) were added to platelet rich rabbit plasma containing 3.8% sodium citrate (1/10 v/v) and incubated at 37 °C for 3 minutes. An aggregating agent, adenosine-5-diphosphate (final concentration 2.5μM), was added thereto and the platelet aggregating potency was measured by an aggregometer.

The result is shown in Table 1. The compound used in the present invention shows a distinct inhibitory effect on platelet aggregation. However control agents, tetramethylpyrazine, ticlopidine and acetyl salicyclic acid have not inhibitory effect.

Table 1

| Compound | Inhibition ratio on platelet aggregation % (mean ± S.E) |
|---|---|
| Present compound | 69.6 ± 5.4 (5)* |
| Tetramethyl pyrazine | 3.0 ± 1.6 (3)* |
| Ticlopidine | -1.2 ± 1.2 (3)* |
| Acetyl salicylic acid | -0.6 ± 0.6 (3)* |

*( ): numbers tested

(2) Effect in vivo:

The agents to be tested were administered orally to male Wistar rats, 5 animals in a group. After one hour, E. coli lipopolysaccharide (5 mg/kg) was injected intraperitoneally and 3 hours thereafter blood samples were collected. The number of platelets in the circulating blood were measured to determine the ratio of platelet numbers at pre- and post-administration of the agents. The tests were twice repeated. The result is shown in Table 2. A marked decrease in platelet numbers is found in the control group. Oral administration of 100mg/kg of the compound used in the present invention inhibits the decrease in platelet numbers, which is comparable to an oral dose of 200 mg/kg of ticlopidine.

Table 2

| Compound | Dose mg/kg p.o. | Number of platelet (%) | |
|---|---|---|---|
| | | Exp. 1 | Exp. 2 |
| Control | — | 27.5 ± 5.4 | 35.1 ± 12.3 |
| Present compound | 100 | 58.2 ± 7.8 | 55.9 ± 14.0 |
| Ticlopidine | 200 | 57.9 ± 12.0 | 63.4 ± 19.4 |

Test example 2

Effect against disseminated intravascular coagulation:

The agents to be tested were administered orally to male Wistar rats, 7 animals in a group, in an amount of 100 mg/kg. After one hour, E. coli lipopolysaccharide (50 mg/kg) was continuously injected intravenously for 4 hours. The control group was given physiological saline intravenously under the same conditions.

Blood was collected immediately after finishing the injection to measure the number of platelets, activated partial thromboplastin time, prothrombin time and amount of fibrinogen. The result is shown in Table 3. In the control group, to which E. coli lipopolysaccharide was injected, marked decreases in the numbers of platelets and amounts of fibrinogen and prolongation of activated partial thromboplastin time and prothrombin time were observed. In the model of experimental disseminated intravascular coagulation , the compound used in the present invention shows significant effect against all factors tested.

### Table 3

| | Platelet number ($\times 10^3/mm^3$) | Activated partial thromboplastin time (s ) |
|---|---|---|
| Control (normal) | 640 ± 58 | 25.5 ± 2.2 |
| Control (patho-model) | 93 ± 26 | 78.6 ± 11.2 |
| Present compound | 177 ± 51 | 48.5 ± 7.4 |

| Prothrombin time (s ) | Fibrinogen (mg/dl) |
|---|---|
| 11.6 ± 0.3 | 17.09 ± 14.4 |
| 19.8 ± 3.2 | 32.7 ± 11.5 |
| 14.3 ± 1.4 | 77.2 ± 13.1 |

Test example 3

Vasodilating activity:

(1) Effect at an intraarterial administration:

Mongrel dogs anesthetized with urethane ( 450 mg/kg iv ) and α-chloralose ( 45 mg/kg iv ) after pretreatment with morphine (1.5 mg/kg, sc) were held in a supine position. Right femoral artery blood was introduced into the left femoral artery through a circulation pump and the left hind-leg was perfused under constant pressure by connecting with Starling's resistance in an exosomatic circulatory system. The prefusion pressure was set slightly higher than the mean blood pressure of each animal and the change in blood flow by intraarterial injection of the test compound was determined.

The result showing specific activity as compared with a value indicating the degree of increase in the amount of blood flow by papaverine ( 30 μg,iv ) as 100% is shown in Table 4. The compound used in the present invention shows distinctly stronger vasodilating activity than that of the control drug, tetramethyl-pyrazine.

### Table 4

| Compound | Dose ($\mu$g, i.a.) | Vasodilating activity % |
|---|---|---|
| Present compound | 100 | 100.7 ± 19.1 * |
| Tetramethylpyrazine | 100 | 19.3 ± 0.9 * |

* mean ± S.E. for 3 animals

(2) Effect at an intravenous administration:

A cuff type probe for measuring blood flow was set in a left vertebral artery, left anterior descending coronary artery, left renal artery and right femoral artery of a mongrel dog anesthetized as above. The blood flow was continuously measured. The blood pressure was monitored through a pressure transducer connected to a cannula inserted in the left femoral artery, and the heart rate was monitored through a tachometer by triggering R-wave of lead II electrocardiogram. The agent to be tested was injected into the left venous cephalica. The result is shown in Fig. 1.

In the figure the increasing effect on blood flow of the compound used in the present invention in dogs is shown. The ordinate indicates the response ratio and the abscissa indicates the intravenous administration dose. The following symbols are used:

●: vertebral arterial blood flow;
▲: coronary arterial blood flow;
■: femoral arterial blood flow;
□: renal arterial blood flow;
○: mean blood pressure;
△: heart rate.

The compound used in the present invention does not affect the renal arterial blood flow and increases the blood flow of vertebral artery, coronary artery and femoral artery. Especially selective effects on the vertebral arterial blood flow and coronary arterial blood flow are observed and so the compound of the present invention is useful for the improvement of brain and heart haemodynamic disorders.

Test Example 4

A protective effect against anoxia:

Male ddY strain mice, 12 animals in a group, were put into an observation chamber filled with 100% carbon dioxide gas. The survival time was observed by signs of apnea. Agents were administered orally 30 or 60 minutes before the test. The result is shown in Table 5.

A significant prolongation in the survival time is observed with any pretreatment of the compound used in the present invention.

Table 5

| Compound | Dose mg/kg p.o. | Pretreatment time (min.) | Survival time (s __) |
|---|---|---|---|
| Control | — | 30 | 29.1 ± 0.5 * |
| Present compound | 200 | 30 | 42.7 ± 1.6 * |
| Control | — | 60 | 31.6 ± 0.6 * |
| Present compound | 200 | 60 | 48.1 ± 1.9 * |

\* mean ± S.E.

Test example 5

Effect against platelet activating factor (PAF) induced shock:

A compound used in the present invention was administered orally to male ddY strain mice, 10 animals in a group, in an amount of 100 mg/kg. After one hour PAF (60 μg/kg) was administered intravenously and the mortality ratio was determined. The result is shown in Table 6 in which shows the protective action of the compound used in the present invention on PAF-induced shock.

Table 6

| | Motality | |
|---|---|---|
| | Experiment 1 | Experiment 2 |
| Control | 9/10 | 10/10 |
| Present compound | 1/10 | 3/10 |

Test example 6

Acute toxicity:

A compound used in the present invention was administered to male ddy mice and male Wister rats to test acute toxicity. The fifty per cent lethal dose was approximately 200 mg/kg in mice. No deaths were observed at an intravenous dose of 100 mg/kg in mice. Rats can only be administered with up to 50 mg/kg of the compound due to the solubility of the compound. No deaths were observed at the dose of 50 mg/kg intravenously. Fifty per cent lethal oral doses were 555 mg/kg for mice and 903 mg/kg for rats.

Test example 7

Cytotoxicity:

To L5178Y murine lymphoblastoma ( $2 \times 10^4$ cells/ml) suspended in a medium (27 ml) comprising Fischer's culture medium and containing 10% bovine serum was added test agent solution (0.3 ml) diluted with culture medium. The mixture was incubated at 37 °C for 18 hours. The growth of cells was determined by color change. Growth inhibition of cells was not observed at concentrations of 20 μg/ml and 100 μg/ml of the compound used in the present invention. Thus no cytotoxicity was observed.

Test example 8

Mutagenicity:

Backward mutation test using microorganisms strains TA100, TA1535, WP2 uvr A, TA98, TA1537 and TA1538 was performed by a method according to Iyer et al. [N.Y.Iyer and W. Syzbalski,Appl. Microbiol., 6: 27 (1958)]. Test agents were diluted at 6 step concentrations of 0.00333 ~ 333 μg/disc at dilution ratio of 10:1. No mutagenesis was found at any concentration of the compound used in the present invention. Also no mutagenesis was observed under metabolic activation conditions using a S-9 mixture.

As clearly illustrated, 2-hydroxy-3-isopropyl-5,6-dimethylpyrazine or non-toxic salt thereof has an inhibitory effect on platelet aggregation, vasodilating activity and protective effect against anoxia with low toxicity. It can be used to improve haemodynamic and metabolic disorders.

The compound used in the present invention can be prescribed per se or in various forms of administration orally or parenterally, such as intramuscularly, subcutaneously, intravenously, rectally or cutaneously. The preferred oral daily dose is from 1 to 600 mg for one man. The composition comprising the compound used in the present invention can be in solid form, such as a tablet, sugar coated tablet, film tablet, hard or soft capsule, trouch, pill, granules and powder, semi-solid form such as a suppository, cataplasm and ointment, or liquid form such as an injection, syrup, inhalant, emulsion and suspension. The compound can be used in the above formulations per se or can be mixed with other components.

The following Examples further illustrate the present invention

Example 1

Injection

2-hydroxy-3-isopropyl-5,6-dimethylpyrazine hydrochloride (10 mg) was dissolved in distilled water for injection (20 ml), adjusted to a concentration of 0.5 mg/ml, sterilized, and filled up to 1 ml in an ampule to prepare the injection.

Example 2

```
Tablet:

2-hydroxy-3-isopropyl-5,6-

dimethylpyrazine hydrochloride        50    mg

lactose                              80    mg

crystalline cellulose                40    mg

magnesium stearate                   0.5   mg

talcum                               29.5  mg
```

Tablets (200 mg/tablet) comprising the above composition were prepared by a dry tabletting method.

Example 3

Drip Infusion:

Injectable liquid formulae containing 2-hydroxy-3-isopropyl-5,6-dimethylpyrazine hydrochloride (10 mg) was diluted in 5% glucose solution (250 ml) to prepare a drip infusion.

## Claims
## Claims for the following Contracting States: DE, FR, IT

1. 2-Hydroxy-3-isopropyl-5,6-dimethylpyrazine or a non-toxic salt thereof for use in a method of treatment of the human or animal body by therapy.

2. A compound according to claim 1 for use in the treatment of a haemodynamic or metabolic disorder.

3. A compound according to claim 2 for use in inhibiting platelet aggregation.

4. A compound according to claim 2 for use as a vasodilator.

5. A compound according to claim 2 for use in protecting against anoxia.

6. A compound according to any one of the preceding claims, which is 2-hydroxy-3-isopropyl-5,6-dimethylpyrazine hydrochloride.

7. Use of 2-hydroxy-3-isopropyl-5,6-dimethylpyrazine or a non-toxic salt thereof in the preparation of a pharmaceutical composition for use in a method of treatment of a haemodynamic or metabolic disorder.

8. A pharmaceutical composition comprising 2-hydroxy-3-isopropyl-5,6-dimethylpyrazine or a non-toxic salt thereof as active ingredient and a pharmaceutically acceptable carrier or diluent.

9. A process for the preparation of a pharmaceutical composition, which process comprises mixing 2-hydroxy-3-isopropyl-5,6-dimethylpyrazine or a non-toxic salt thereof with a pharmaceutically acceptable carrier or diluent, the said mixing being effected under sterile conditions and/or the obtained mixture being sterilised.

10. Non-toxic salts of 2-hydroxy-3-isopropyl-5, 6-dimethylpyrazine.

11. A process for the preparation of a non-toxic salt of 2-hydroxy-3-isopropyl-5,6-dimethylpyrazine, which process comprises condensing 2,3-butanedione with valineamide in the presence of a tertiary amine and converting the resultant 2-hydroxy-3-isopropyl-5,6-dimethylpyrazine into a non-toxic salt thereof.

**Claims for the following Contracting State: ES**

1. A process for the preparation of a pharmaceutical composition, which process comprises mixing 2-hydroxy-3-isopropyl-5,6-dimethylpyrazine or a non-toxic salt thereof with a pharmaceutically acceptable carrier or diluent, the said mixing being effected under sterile conditions and/or the obtained mixture being sterilised.

2. A process according to Claim 1 wherein the salt is the hydrochloride.

3. A process for the preparation of a non-toxic salt of 2-hydroxy-3-isopropyl-5,6-dimethylpyrazine, which process comprises condensing 2,3-butanedione with valineamide in the presence of a tertiary amine and converting the resultant 2-hydroxy-3-isopropyl-5,6-dimethylpyrazine into a non-toxic salt thereof.

4. A process according to Claim 3 wherein the salt is the hydrochloride.

**Revendications**
**Revendications pour les Etats contractants suivants: DE, FR, IT**

1. 2-hydroxy-3-isopropyl-5,6-diméthylpyrazine ou un de ses sels non toxiques pour son utilisation dans un procédé de traitement thérapeutique du corps humain ou d'un animal.

2. Composé selon la revendication 1 pour son utilisation dans le traitement d'une maladie hémodynamique ou métabolique.

3. Composé selon la revendication 2, pour son utilisation pour inhiber l'aggrégation des plaquettes.

4. Composé selon la revendication 2 pour son utilisation en tant que vasodilatateur.

5. Composé selon la revendication 2 pour son utilisation pour protéger contre l'anoxie.

6. Composé selon l'une des revendications précédentes, qui est le chlorure de 2-hydroxy-3-isopropyl-5,6-méthylpyrazine.

7. Utilisation de la 2-hydroxy-3-isopropyl-5,6-méthylpyrazine ou d'un de ses sels non toxiques pour préparer une composition pharmaceutique destinée à être utilisée dans une méthode de traitement d'une maladie hémodynamique ou métabolique.

8. Composition pharmaceutique contenant la 2-hydroxy-3-isopropyl-5,6-diméthyl-pyrazine ou un de ses sels non toxiques en tant qu'ingrédient actif et un véhicule ou diluant pharmaceutiquement acceptable.

9. Procédé pour préparer une composition pharmaceutique, consistant à mélanger la 2-hydroxy-3-isopropyl-5,6-diméthylpyrazine ou un de ses sels non toxiques avec un véhicule ou un diluant pharmaceutiquement acceptable, ce mélange s'effectuant dans des conditions stériles et/ou le mélange obtenu étant stérilisé.

10. Sels non toxiques de la 2-hydroxy-3-isopropyl-5,6 diméthylpyrazine.

11. Procédé pour préparer un sel non toxique de la 2-hydroxy-3-isopropyl-5,6-diméthylpyrazine, consistant à condenser la 2,3-butanedione avec l'amide de la valine en présence d'une amine tertiaire et à convertir la 2-hydroxy-3-isopropyl-5,6-diméthylpyrazine en un sel non toxique.

**Revendications pour l'Etat contractant suivant: ES**

1. Procédé pour préparer une composition pharmaceutique, consistant à mélanger la 2-hydroxy-3-isopropyl-5,6-diméthylpyrazine ou un de ses sels non toxiques avec un véhicule ou un diluant pharmaceutiquement acceptable, ce mélange s'effectuant dans des conditions stériles et/ou le mélange

9

obtenu étant stérilisé.

2. Procédé selon la revendication 1, dans lequel le sel est le chlorure.

3. Procédé pour préparer un sel non toxique de la 2-hydroxy-3-isopropyl-5,6-diméthylpyrazine, consistant à condenser la 2,3-butanedione avec l'amide de la valine en présence d'une amine tertiaire et à convertir la 2-hydroxy-3-isopropyl-5,6-diméthylpyrazine en un sel non toxique.

4. Procédé selon la revendication 3, dans lequel le sel est le chlorure.

**Patentansprüche**
**Patentansprüche für folgende Vertragsstaaten: DE, FR, IT**

1. 2-Hydroxy-3-isopropyl-5,6-dimethylpyrazin oder ein nichttoxisches Salz davon zur Verwendung in einem Verfahren zur therapeutischen Behandlung des menschlichen oder tierischen Körpers.

2. Verbindung nach Anspruch 1 zur Behandlung einer haemodynamischen oder metabolischen Krankheit.

3. Verbindung nach Anspruch 2 zur Inhibierung der Platelet-Aggregation.

4. Verbindung nach Anspruch 2 zur Verwendung als Vasodilatator.

5. Verbindung nach Anspruch 2 zum Schutz gegen Anoxie.

6. Verbindung nach einem der vorhergehenden Ansprüche, dadurch gekennzeichnet, daß sie 2-Hydroxy-3-isopropyl-5,6-dimethylpyrazinhydrochlorid ist.

7. Verwendung von 2-Hydroxy-3-isopropyl-5,6-dimethylpyrazin oder einem nicht-toxischen Salz davon zur Herstellung einer pharmazeutischen Zusammensetzung zur Verwendung in einem Verfahren zur Behandlung einer haemodynamischen oder metabolischen krankheit.

8. Pharmazeutische Zusammensetzung, dadurch gekennzeichnet, daß sie 2-Hydroxy-3-isopropyl-5,6-dimethylpyrazin oder ein nichttoxisches Salz davon als aktiven Bestandteil und einen pharmazeutisch annehmbaren Träger oder Verdünner enthält.

9. Verfahren zur Herstellung einer pharmazeutischen Zusammensetzung, dadurch gekennzeichnet, daß man 2-Hydroxy-3-isopropyl-5,6-dimethylpyrazin oder ein nicht-toxisches Salz davon mit einem pharmazeutischen annehmbaren Träger oder Verdünner mischt, wobei die Mischung unter sterilen Bedingungen durchgeführt wird und/-oder die erhaltene Mischung sterilisiert wird.

10. Nicht-toxisches salze von 2-Hydroxy-3-isopropyl-5,6-dimethylpyrazin.

11. Verfahren zur Herstellung eines nicht-toxischen Salzes von 2-Hydroxy-3-isopropyl-5,6-dimethylpyrazin, dadurch gekennzeichnet, daß man 2,3-Butandion mit Valinamid in Gegenwart eines tertiären Amins kondensiert und das resultierende 2-Hydroxy-3-isopropyl-5,6-dimethylpyrazin in ein nicht-toxisches Salz davon überführt.

**Patentansprüche für folgenden Vertragsstaat: ES**

1. Verfahren zur Herstellung einer pharmazeutischen Zusammensetzung, dadurch gekennzeichnet, daß man 2-Hydroxy-3-isopropyl-5,6-dimethylpyrazin oder ein nicht-toxisches Salz davon mit einem pharmazeutiscn annehmbaren Träger oder verdünner mischt, wobei die Mischung unter sterilen Bedingungen durchgeführt wird und/-oder die erhaltene Mischung sterilisiert wird.

2. Verfahren nach Anspruch 1, dadurch gekennzeichnet, daß das Salz das Hydrochlorid ist.

3. Verfahren zur Herstellung eines nicht-toxischen Salzes von 2-Hydroxy-3-isopropyl-5,6-dimethylpyrazin, dadurch gekennzeichnet, daß man 2,3-Butandion mit Valinamid in Gegenwart eines tertiären Amins kondensiert und das resultierende 2-Hydroxy-3-isopropyl-5,6-dimethylpyrazin in ein nicht-toxisches Salz davon überführt.

4.  Verfahren nach Anspruch 3, dadurch gekennzeichnet, daß das Salz das Hydrochlorid ist.